# EUROPEAN PATENT SPECIFICATION

(11) **EP 1 838 656 B1**
(45) Date of publication and mention of the grant of the patent: **04.11.2015**
(21) Application number: 06700787.2
(22) Date of filing: 13.01.2006
(51) Int. Cl.: C07C 67/02, C07C 67/08

(54) **PROCESS FOR PREPARING AN ACETOGLYCERIDE COMPOSITION COMPRISING HIGH AMOUNTS OF MONOACETYL MONOACYL GLYCERIDES**
VERFAHREN ZUR HERSTELLUNG EINER ACETOGLYCERIDZUSAMMENSETZUNG ENTHALTEND EINEN HOHEN ANTEIL AN MONOACETYLMONOACYLGLYCERIDE
PROCÉDÉ POUR LA PRÉPARATION D'UNE COMPOSITION D'UN ACÉTOGLYCÉRIDE RICHE EN MONOACETYLMONOACYLGLYCERIDE

(30) Priority: 13.01.2005 EP 05075100
(43) Date of publication of application: 03.10.2007
(73) Proprietor: Paramelt B.V., 1704 RJ Heerhugowaard (NL)
(72) Inventor: DIJKSTRA, Albert Jan, F-47210 St. Eutrope-de-born (FR); BROCKE, Petrus Conradus, NL-1705 NS Heerhugowaard (NL); WOLDHUIS, Jan, NL-1827 MG Alkmaar (NL)
(74) Representative: Nederlandsch Octrooibureau
(86) International application number: PCT/NL2006/050009
(87) International publication number: WO 2006/091096

(56) References cited:
- US-A- 2 764 605
- US-A- 5 434 278

## Description

### FIELD OF THE INVENTION

The present invention relates to a process for preparing an acetoglyceride composition comprising high amounts of monoacetyl monoacyl glycerides and to the acetoglyceride compositions thus obtained.

### BACKGROUND OF THE INVENTION

Acetoglycerides constitute a class of compounds comprising a mixture of carboxylic acid esters of glycerol (1,2,3-propanetriol) containing one or two long chain fatty acid moieties (in this patent application long chain fatty acid moieties are generally referred to as acyl moieties, wherein the fatty acid moieties have a linear, saturated or unsaturated, C₁₄ - C₂₄ alkyl chain), one or two short chain carboxylic acid moieties (according to this patent application short chain carboxylic acid moieties are moieties having a linear or branched, preferably linear, saturated C₁ - C₄ alkyl group), e.g. acetyl groups, and possibly one free hydroxy group. This class of compounds was discovered by *Wilhelm Normann* in 1920 and is disclosed in German Patent Specification DE C 417.215. In this patent specification, a high temperature interesterification process between tristearin and triacetin (1,2,3-propanetriol triacetate) is used to prepare the acetoglycerides and it is noted that the interesterification product is quite hard but not brittle and becomes waxy on kneading.

The physical properties of acetoglycerides have been found to depend strongly upon the degree of unsaturation of the long chain fatty moieties, the free hydroxy group content and the content of diacyl glycerides, i.e. glycerides having two long chain fatty acid moieties. As disclosed in European Patent Application EP A 0.679.337 (equivalent to US 5.516.536), an acetoglyceride composition can be used as a coating composition to provide a protective coating for cheese. The acetoglyceride composition according to EP A 0.679.337 comprises acetic acid esters of monoacyl glycerides, the acyl group being essentially derived from long-chain fatty acids predominantly comprising stearic and behenic acid. The acetic acid esters of the monoacyl glycerides contain about 40 to 57 wt.% diacetic acid esters (diacetyl monoacyl glycerides) and about 40 to 50 wt.% monoacetic acid esters (monoacetyl monoacyl glycerides).

As explained above, where the short chain carboxylic acid moieties are acetyl moieties, acetoglycerides comprise the following group of compounds: monoacetyl diacyl glycerides, diacetyl monoacyl glycerides and monoacetyl monoacyl glycerides. In this patent application, the following definitions are therefore used to identify the various compounds belonging to the group of acetoglycerides and other compounds:
- Monoacetyl diacyl glycerides: glycerol esters having two acyl groups and one acetyl group;
- Diacyl glycerides: glycerol esters having two acyl groups and one free hydroxy group;
- Diacetyl monoacyl glycerides: glycerol esters having one acyl group and two acetyl groups;
- Monoacetyl monoacyl glycerides: glycerol esters having one acyl group, one acetyl group and one free hydroxy group;
- Monoacyl glycerides: glycerol esters having one acyl group and two free hydroxy groups.

Obviously, compounds such as monoacetyl diacyl glycerides can occur in different structures, i.e. 1,3-propanetriol-distearate-2-acetate and 1,2-propanetriol-distearate-3-acetate, as will be apparent to the person skilled in the art.

According to EP A 0.679.337, the coating compositions can be produced by reacting distilled monoacyl glycerides having a content of monoester above 90% with acetic anhydride at a temperature in the range of 100°C to 150°C, simultaneously removing the by-product acetic acid formed. This production route has several disadvantages in that it uses expensive raw materials and leads to undesired by-products. The preparation of the monoacyl glycerides used as raw material involves allowing glycerol to react with a triacyl glyceride oil in a glycerolysis process leading to a mixture of non-reacted triacyl glyceride oil, diacyl glycerides, monoacyl glycerides and free glycerol. Such a reaction mixture contains at most about 60 % monoacyl glycerides by weight due to the limited solubility of glycerol in the reaction mixture. Consequently, obtaining a monoacyl glyceride product having a content of monoacyl glyceride above 90% involves separation steps, e.g. molecular distillation, of the reaction mixture, which is an expensive process.

US 1.558.299 discloses several processes for the preparation of acetoglycerides comprising monoacetyl diacylglycerides and diacetyl monoacyl glycerides. A preferred process comprises the acid catalysed conversion of glycerol, glacial acetic acid and fatty acids in a molar ratio of about 1.3 : 5.0 : 1 at a relatively low temperature, i.e. below 170°C. Consequently, this process is not directed to the preparation of monoacetyl monoacyl glycerides.

US 2.615.160 discloses a process for the preparation of a fatty composition consisting essentially of diacetyl monoacyl glycerides, wherein a mixture of triacetin and a conventional fat is contacted at a relatively low temperature with a basic interesterification catalyst. The end product of this process still contains some triacetin and some of the conventional fat used as starting materials. All unreacted triacetin is first removed from the end product by steam stripping under reduced pressure, where after a fraction consisting predominantly of high molecular diacetyl monoacyl glycerides is isolated by steam stripping under reduced pressure at increased temperature. The residue is largely a mixture of monoacetyl diacyl glycerides and the conventional fat used as the starting material. If desired, this mixture can be recycled into the process.

US 2.764.605 discloses a process for the preparation of a monoacetyl monoacyl glyceride wherein a triacyl glyceride, glycerol and an acetylated glycerol, preferably triacetin, are contacted with a basic interesterification catalyst at relatively high temperatures, i.e. at least 180°C and in particular from 200° to 280°C. US 2.764.605 further discloses that the degree of acetylation of the triacyl glyceride is dependent upon (a) the molar ratio of acetylated glycerol and glycerol and (b) the degree of acetylation of the acetylated glycerol and that the conversion of the triacyl glyceride depends upon the combined amount of glycerol and acetylated glycerol. To isolate the desired product, a two stage vacuum distillation process is used with a first stage operated at 135°C to remove glycerol and acetins as a top product and a second stage at 165 - 185°C to isolate the monoacetyl monoacyl glyceride as a top product. The residue comprises monoacetyl diacyl glycerides and triacyl glycerides. The molar ratios of glycerol, lard and triacetin used in Examples 1, 3 and 4 are listed in the table below. Since in this patent application reference is made to molar ratios of long chain fatty acid moieties LCFAM, glycerol moieties GM and short chain carboxylic acid moieties, preferably acetyl moieties AC (see below; in this patent application reference is in general made to "acetyl moieties" whereas this term encompasses short chain carboxylic acid moieties as defined above), the table provides a conversion of the molar ratios of glycerol, lard and triacetin:

| Examples US 2.764.605 | Glycerol: Lard : Triacetin | GM : LCFAM : AC |
|---|---|---|
| Example 1 | 1.00 : 0.82 : 2.00 | 1 : 0.64 : 1.57 |
| Example 3 | 1.00 : 0.33 : 1.00 | 1 : 0.42 : 1.29 |
| Example 4 | 1.00 : 0.51 : 2.00 | 1 : 0.44 : 1.71 |

However, also this process is expensive because of the high vacuum required and the high energy consumption since the monoacetyl monoacyl glyceride is isolated as a top product.

US 2.808.421 discloses a process for the preparation of a mixture of monoacetyl diacyl glycerides and diacetyl monoacyl glycerides wherein a triacyl glyceride and e.g. triacetin are reacted under anhydrous conditions in the presence of a basic interesterification catalyst at a temperature of at least 180°C. Isolation of the desired end product is achieved by distillation techniques. Again, it is worth noting that the desired product is obtained as top product which is accompanied by a relatively high energy consumption.

US 5.434.278 and US 5.662.953 disclose a solvent-free process for the preparation of a mixture of diacetyl monoacyl glycerides and monoacetyl diacyl glycerides wherein triacyl glycerides and triacetin are reacted in molar ratios of 1 : 1 to 1 : 15 at a temperature of about 100° to about 150°C in the presence of a catalyst.

### OBJECTS OF THE INVENTION

It is an object of the invention to overcome the disadvantages of the processes according to the prior art.

It is a further object of the invention to prepare acetoglycerides with a content of monoacetyl diacyl glycerides of less than 20% or even less than 15%.

It is a further object of the invention to use simple and easily accessible starting materials.

It is also an object of the invention to avoid by-products by enabling these to be recycled.

It is yet another object of the invention to avoid the use of expensive, very high vacuum equipment.

Yet another object of the invention is to provide a process having a relatively low energy consumption.

Further objects and advantages of the invention will become apparent from the description hereinafter.

### SUMMARY OF THE INVENTION

Whereas the glycerolysis process of triacyl glycerides, which leads to a mixture of free glycerol, monoacyl glycerides, diacyl glycerides and triacyl glycerides, is limited in the monoacyl glyceride content of the glycerolysis product because of the limited solubility of glycerol in said glycerolysis product, it has surprisingly been found that the presence of acetic acid and its esterification products in said glycerolysis product increases the glycerol solubility in said glycerolysis product and thus allows the molar ratio of (monoacetyl monoacyl glycerides + diacetyl monoacyl glycerides = acetoglycerides having one long chain fatty acid moiety) in said product to (diacyl glycerides + monoacetyl diacyl glycerides = glycerides having two long chain fatty acid moieties) to be raised at will.

Accordingly, the present invention provides a process for preparing a acetoglyceride composition comprising a high amount of monoacetyl monoacyl glycerides comprising the steps of:
(a) providing a reaction mixture comprising long chain fatty acid moieties, glycerol moieties and short chain carboxylic acid moieties, preferably acetyl moieties;
(b) adding a catalyst;
(c) allowing this reaction mixture to approach or reach equilibrium and optionally shift the position of this equilibrium by removing the most volatile component or components from said reaction mixture;
(d) inactivating and/or removing the catalyst from the reaction mixture;
(e) purifying the reaction mixture;
wherein the purification step (e) comprises a separation of said reaction mixture into an acetoglyceride fraction and one or more non-acetoglyceride fractions and that at least one of those non-acetoglyceride fractions is recycled by being incorporated into the reaction mixture provided in step (a);
wherein the molar ratio of the long-chain fatty acid moieties and the glycerol moieties in the reaction mixture provided in step (a) is between 1 : 8 and 1 : 3; and
wherein the molar ratio of the short chain carboxylic acid moieties, preferably acetic acid moieties, and the glycerol moieties in the reaction mixture provided in step (a) is between 1.5 : 1 and 3 : 1.

### DETAILED DESCRIPTION OF THE INVENTION

According to a preferred embodiment of the process according to the invention, the molar ratio of the long-chain fatty acid moieties and the glycerol moieties in the reaction mixture provided in step (a) is between 1 : 8 and 1 : 3 whereas the molar ratio of the short chain carboxylic acid moieties, preferably acetic acid moieties, and the glycerol moieties in the reaction mixture provided in step (a) is between 1.5 : 1 and 3 : 1.

According to the present invention, it is further preferred that the acetoglyceride composition comprises a relatively large number of free hydroxy groups. Accordingly, it is therefore preferred that the molar ratio of (i) the sum of long-chain fatty acid moieties and short chain carboxylic acid moieties and (ii) the glycerol moieties does not exceed 2.9 : 1, preferably does not exceed 2.7 : 1.

According to the process of the invention, acetoglycerides are prepared by providing a reaction mixture comprising long chain fatty acid moieties, glycerol moieties and acetyl moieties. As described above, long chain fatty acid moieties are in this application generally referred to as acyl moieties, wherein the long chain fatty acid moieties have a linear, saturated or unsaturated, C₁₄ - C₂₄ alkyl chain. Preferably, the average chain length of the long chain fatty acid moieties is in the range of 17.5 - 21.5 carbon atoms, wherein the "average chain length" is defined as the average resulting from dividing the total number of carbon atoms in the long chain fatty acid moieties present as esterified and free moieties with the total number of these moieties (cf. US 5.516.536 for this definition). The long chain fatty acid moieties may originate from various starting materials, i.e. triacyl glycerides, diacyl glycerides, monoacyl glycerides, free fatty acids and mixtures thereof.

The glycerol moieties comprised in the reaction mixture provided in step (a) of the process according to the invention may also originate from various starting materials including triacyl glycerides, diacyl glycerides, monoacetyl diacyl glycerides, monoacyl glycerides, diacetyl monoacyl glycerides, monoacetyl, diacetyl and triacetyl esters of glycerol (generally referred to as "acetins"), free glycerol and mixtures thereof. However, the glycerol moieties will preferably originate from triacyl glycerides, free glycerol and acetins, more preferably from triacyl glycerides, free glycerol and the non-acetoglyceride fraction resulting from the separation in purification step (e) of the process according to the invention as will be explained in more detail below.

The acetyl moieties comprised in the reaction mixture provided in step (a) of the process according to the invention may also originate from various starting materials including acetic acid, acetic acid anhydride, monoacetyl diacylglycerides, diacetyl monoacylglycerides, monoacetyl, diacetyl and triacetyl esters of glycerol ("acetins") and mixtures thereof. Preferably, the acetyl moieties are provided by the various acetins as for instance provided by the non-acetoglyceride fraction resulting from the separation in purification step (e) of the process according to the invention. In industrial practice, the acetyl moieties are most preferably provided by glacial acetic acid, by recycling acetic acid when this is formed as a by-product and/or by recycling the non-acetoglyceride fraction resulting from step (e). In general this is cheaper than purchasing acetins and using these compounds as a source of acetyl moieties.

Accordingly, the compounds to be included in the reaction mixture as provided in step (a) of the process according to the invention are preferably: triacyl glycerides, preferably triacyl glycerides containing the long chain fatty acids aimed for in the acetoglycerides, free glycerol, glacial acetic acid and the non-acetoglyceride fraction resulting from the separation step (e). In this way, the raw materials used in the process according to the invention are easily accessible and therefore the cheapest and besides, the by-product resulting from the separation step is fully utilised so that the process according to the invention does not lead to any organic by-products that must be disposed of.

The molar ratios of the long chain fatty acid moieties, glycerol moieties and acetyl moieties are selected within the specified ranges and in accordance with the acetoglyceride composition aimed for. If for instance the desired product is an acetoglyceride with about 20 % by weight of (monoacetyl diacyl glycerides + diacyl glycerides) and about 80 % by weight of (monoacetyl monoacyl glycerides + monoacyl glycerides), the long chain fatty acid to glycerol molar ratio in the process according to the invention has to be about 1 : 3. Similarly, if the acetylation degree aimed for is for instance about 70%, the acetic acid to glycerol molar ratio in the process according to the invention has to be about 2:1.

The nature of the long chain fatty acid moieties to be included in the reaction mixture is not critical for the process according to the invention; they solely determine the acetoglyceride properties. Accordingly, vegetable oils such as soybean oil or low erucic acid rapeseed oil can be used if a high content of fatty acids with a chain length of 18 carbon atoms in the acetoglyceride is deemed to be desirable. If fatty acids having a longer chain are needed, fish oil or high erucic acid rapeseed oil can be used when providing the reaction mixture in step (a) of the process according to the invention. The long chain fatty acid moieties can be provided by employing starting materials or mixtures thereof as described above. Suitable examples are triacyl glycerides, diacyl glycerides, monoacyl glycerides, free fatty acids and mixtures thereof. Alternatively, the starting materials can also be provided by the product of a fractionation process as for instance a fractional vacuum distillation process in the case of free fatty acids or a fractional crystallisation process in the case of triacyl glycerides or fatty acids. Obviously and if desired, the fatty acids can be partially or fully hydrogenated but the process sequence in which non-hydrogenated triacyl glycerides are used in the preparation of the acetoglycerides and in which the acetoglycerides thus obtained are subsequently hydrogenated also falls within the scope of the present invention.

If a basic catalyst is to be added in step (b) of the process according to the invention, the reaction mixture provided in step (a) must be devoid of any free acids such as free fatty acids and free acetic acid since these free acids would inactivate the basic catalyst. Consequently, according to the invention the reaction mixture comprising long chain fatty acid moieties, glycerol moieties and short chain carboxylic acid moieties is then essentially free from components that are capable of deactivating the catalyst. Accordingly, the fatty acid moieties to be provided in step (a) then comprise triacyl glycerides, diacyl glycerides and monoacyl glycerides. In addition the reaction mixture is preferably devoid of any water and is therefore preferably dried before the basic catalyst is added. Effective drying can for example be achieved by spraying the warm reaction mixture to be dried in vacuo.

The basic catalyst to be used in the process according the invention is selected from the group of alkali metals, their hydrides, amides and alcoholates, wherein the alcoholates are derived from linear or branched C₁ - C₄ alcohols. Sodium or potassium methanolate or ethanolate are preferred catalysts for the process according to the invention. Normal interesterification temperatures can be used for the process according to the invention. The lowest temperature that can be used is the temperature at which the reaction mixture is fully molten. This temperature is generally about 70°C or higher. However, it is preferred that the reaction temperature should not exceed 100°C.

The use of an acidic catalyst allows the reaction mixture to include free acids such as free long chain fatty acids and acetic acid, and water. Since water is preferably removed when the reaction mixture is allowed to approach equilibrium in step (c) of the process according to the invention, it is preferred that when an acidic catalyst is used that the temperature is in the range of 120 - 150°C during said step (c).

The acidic catalyst to be added in step (b) of the process according to the invention has preferably a pKₐ-value below 4. Acetic acid itself has a pKₐ-value of 4.76 and fatty acids have pKₐ-values of about 5. Accordingly, these rather weak acids require high temperatures for the reaction in step (c) of the process according to the invention to proceed. Such high temperatures lead often to side-reactions so that lower temperatures in the range of 120 - 150°C are preferred which, however, requires the use of a more acidic catalyst. Inorganic acids like phosphoric acid (pKₐ = 2.16) has been found to be an effective catalyst and organic acids like C₁ - C₁₀ hydrocarbyl sulphonic acids (the hydrocarbyl group is preferably a linear, branched or cyclic, saturated or (partially) unsaturated group) such as toluenesulphonic acid and especially methanesulphonic acid are also, highly effective acidic catalysts when the process according to the present invention is conducted within the temperature range of 120 - 150°C. Sulphuric acid itself (pKₐ < 2), although effective as a catalyst, tends to cause the reaction mixture to discolour and is therefore not recommended. Consequently, according to the invention, the acidic catalyst is preferably selected from the group consisting of acids having a pKₐ in the range of 2 - 4.

In step (c) of the process according to the invention, the reagents are allowed to react and approach or reach equilibrium. This requires heating the reaction mixture to a temperature of preferably around 130 °C at atmospheric pressure. Heating to a higher temperature is also possible and has the advantage that the equilibrium is reached more rapidly but then this requires superatmospheric pressure to retain the acetic acid within the reaction mixture.

As will be apparent to the person skilled in the art, the reaction mixture will contain free long chain fatty acids, acetic acid, free glycerol and water, and also mono-, di- and triacyl glycerides comprising one or more acetyl groups. These compounds form usually a single phase. Although two phases or at least some cloudiness has sometimes been observed at the start of the reaction, this has disappeared during the course of the reaction towards equilibrium, especially when this equilibrium has been shifted by the removal of the most volatile compound, said most volatile compound being water. However, the water will obviously also contain minor quantities of acetic acid.

Water is an essential reagent in the acid catalysed interesterification reaction, so some water must be present for the various reactions to proceed. Moreover, the amount of water present in the reaction mixture determines the amount of free acids present in this mixture. Since this mixture will subsequently be separated in step (e) of the process according to the invention to obtain the acetoglyceride composition and because of the affinity of free long chain fatty acids for the acetoglyceride fraction (this fraction is more hydrophobic than the non-acetoglyceride fraction) and the subsequent separation of the acetoglyceride composition, it has been found to be advantageous to retain some water in the reaction mixture during the start of the reaction (c) and to remove it gradually by (azeotropic) distillation as and when the equilibrium is being reached. Removing the water will shift the various equilibria concerned and lower the free long chain fatty acid content of the final reaction mixture which will facilitate its subsequent purification by separation.

The gradual removal of water can be easily realised by raising the temperature of the reaction mixture to for example 150 °C or even higher. Since the water to be removed by distillation is a by-product to be discarded, it should preferably contain as little acetic acid as possible. Accordingly, a small fractionation column can be used to prevent acetic acid from being lost from the reaction mixture. However, retaining all acetic acid in said mixture is not essential and the process in which water and some acetic acid distil from the mixture is also within the scope of the invention.

As will be obvious to the person skilled in the art, it is preferred to perform the process according to the invention under essentially oxygen free conditions, e.g. under a nitrogen blanket or under vacuum. When oxygen is present, the products may discolour which may necessitate an additional discolouration step.

In step (d) of the process according to the invention, the catalyst is inactivated and/or removed from the reaction mixture, e.g. by washing the reaction mixture with water. This can be readily achieved by the addition of a small amount of water, preferably 2 - 5 wt.%, based on the total weight of the reaction mixture. If the catalyst is a basic catalyst, the basic catalyst will be inactivated and the inactivation product or products thereof will dissolve. If the catalyst is an acidic catalyst, the acidic catalyst will also dissolve. The temperature of inactivation is preferably below 100°C which means that the temperature of the reaction mixture may have to be lowered before water addition. The water containing the acidic catalyst is preferably separated from the organic reaction product before this is being purified in step (e) of the process according to the invention.

The water used during the catalyst inactivation step (d) will also dissolve the reaction mixture constituents such as acetic acid, glycerol and the acetins. In fact, water washing is an effective means to separate the reaction mixture into a water-soluble non-acetoglyceride fraction and a water-insoluble acetoglyceride fraction. Consequently, according to the invention the purification step (e) may comprise a washing step with water. To this end, the reaction mixture is brought to a temperature below 100°C and mixed with water which has preferably been demineralised since it may have to be removed by evaporation in a later stage; steam condensate has been found to be highly appropriate.

It has surprisingly been found that the use of water comprising some free short chain fatty acid, preferably free acetic acid, in the washing step has a beneficial effect upon the separation into a water-soluble non-acetoglyceride fraction and a water-insoluble acetoglyceride fraction. In particular, the extraction of acetins, in particular triacetins, to the non-acetoglyceride fraction proceeds more efficient when the water comprises 10 - 30 wt.%, more preferably 15 - 25 wt.% of free short chain fatty acid, preferably acetic acid, based on the total weight of the water.

The water-washing step can be carried out in several ways. In a batch process, water can be added to the reactor after its contents have been cooled down, for instance by spraying it across the surface, and then allowing the wash water to settle in the bottom of the reactor, from where it can conveniently be drained. Continuous processes can use in-line mixing systems and centrifugal separators for the water washing operation but mixer-settlers have also been found to be effective. Because the water resulting from the water washing will be evaporated to recuperate its non-aqueous constituents to enable these to be recycled, the amount of water is preferably minimised for example by two or more successive wash steps in the case of the batch process and counter-current washing in case of the continuous process. Consequently, the purification step (e) involves a separation of the reaction mixture into a water-insoluble acetoglyceride fraction and a water-soluble non-acetoglyceride fraction.

According to the invention, it is preferred that the non-acetoglyceride fraction or fractions are substantially and more preferably completely recycled to the process according to the invention by being incorporated into the reaction mixture provided in step (a).

Another means of separation during the purification step (e) applicable to the process according to the invention involves distillation of the reaction mixture. This process is effective since the acetoglyceride compositions constitute the least volatile fraction of said mixture so that it can be isolated as bottom product. Accordingly, the acetoglyceride composition is the distillation residue, and the non-acetoglyceride fraction is the distillate. The distillation is preferably carried out under vacuum to avoid overheating of the still bottoms. Accordingly, the distillative separation step (e) can be combined with a final purification of the acetoglyceride composition by switching the distillation process to a vacuum stripping process employing for instance steam and removing the last traces of the volatiles; this combination may require prior bleaching of the reaction mixture. Hence, according to a preferred embodiment of the present invention, in the purification step (e) the reaction mixture is separated into an acetoglyceride fraction and a non-acetoglyceride fraction, wherein the latter is more volatile than the former thereby enabling the isolation of the acetoglyceride fraction e.g. as a bottom product when the separation is carried out by distillation.

Bleaching conditions for the acetoglyceride compositions prepared by the process according to the invention do not differ significantly from the conditions used for edible oils and fats. Consequently, 0.2 - 2.0 wt% of bleaching earth, which may be activated or not, is mixed under moderate vacuum with the acetoglyceride composition at a temperature of 80 - 100 °C for a period of 15 - 30 minutes, whereafter the bleaching earth is removed by filtration. If the acetoglyceride composition has to be hydrogenated, this can be done either before the composition is bleached. However, it is preferred that the fraction is first bleached and only then hydrogenated. This necessitates a second bleaching treatment but saves on hydrogenation catalyst usage.

Apart from bleaching earth, it may be advantageous to use activated carbon as absorbent during the bleaching process, especially if the reaction mixture has become discoloured by the action of the acidic catalyst. The use of a mixture of bleaching earth and activated carbon can also be considered.

Deodorisation or steam stripping is preferably carried out at elevated temperatures of 220 - 250°C at a pressure of around 3 mbar (0.3 kPa) while using 0.5 - 1.5 wt% stripping steam or other inert stripping medium such as for instance nitrogen. These process conditions will not only remove any long chain fatty acids and acetins that have not been removed during the water washing stage but will also remove malodorous compounds and thus ensure a bland tasting acetoglyceride composition.

If purification step (e) has been effected by distillation, the distillate is preferably recycled as such by incorporating it into the reaction mixture provided in step (a) of the process according to the invention. If step (e) involves a water-washing process, the non-acetoglyceride fraction ends up as a solution in water. Consequently, most of the water is preferably removed from this solution by evaporation before the non-acetoglyceride fraction is recycled into the reaction mixture provided in step (a) of the process according to the invention. However, if an acidic catalyst is used, the non-acetoglyceride fraction may contain some residual water before being recycled since water is produced during the reaction which water may subsequently be removed anyway. This means that the removal process of the water from the non-acetoglyceride fraction can focus on a pure distillate/effluent rather than on an anhydrous residue.

The deodoriser distillate can also be recycled by its incorporation into the reaction mixture to be provided in step (a) of the process according to the invention. If this deodoriser distillate results from the steam refining of the acetoglyceride composition resulting from the separation step (e) by water washing, its weight will generally fully justify its being recycled. On the other hand if its weight is only small, and its smell rather strong, it may well be advisable to discard this deodoriser distillate as a purge for malodorous compounds present in the raw materials or formed during the process according to the invention.

The process according to the invention can also be combined with prior art processes. Accordingly, the process according to the invention can be used to prepare acetoglyceride compositions having a rather low degree of acetylation and in a subsequent reaction said acetoglyceride compositions can then be further acetylated, e.g. by allowing them to react with acetic anhydride. The acetic acid liberated during this acetylation as by-product can then be recycled by incorporation into the reaction mixture provided in step (a) of the process according to the invention.

According to a preferred embodiment of the present invention, the process for preparing an acetoglyceride composition comprising a high amount of monoacetyl monoacyl glyceride comprises the steps of:
(a) providing a reaction mixture comprising a triacyl glyceride, a short chain carboxylic acid, preferably acetic acid, and glycerol, wherein the molar ratio of triacyl glyceride to glycerol is within the range of 0.043 : 1 to 0.125 : 1 and the molar ratio of the short chain carboxylic acid to glycerol is within the range of 1.565 : 1 to 3.375 : 1;
(b) optionally, but preferably adding an esterification catalyst;
(c) heating the reaction mixture of step (b) to approach or reach equilibrium and optionally shift the position of this equilibrium by removing the most volatile component or components from said reaction mixture;
(d) inactivating and/or removing the catalyst from the reaction mixture; and
(e) purifying the reaction mixture,
wherein the purification step (e) comprises a separation of said reaction mixture into an acetoglyceride fraction and one or more non-acetoglyceride fractions and that at least one of those non-acetoglyceride fractions is recycled by being incorporated into the reaction mixture provided in step (a).

The molar ratio ranges of triacyl glyceride (TCA) to glycerol (GLY) and of short chain carboxylic acid (SCCA) to glycerol (GLY) comply with the molar ratio ranges of long chain fatty acid moieties (LCFAM) to glycerol moieties (GLYM) and short chain fatty acid moieties (SCFAM) as specified in this patent application. For example, a molar ratio of LCFAM to GLYM of 1 : 8 and a molar ratio of SCFAM to GLYM of 3 : 1 equals a molar ratio of LCFAM : GLY : SCFAM = 1 : 8 : 24 (or 3 : 24 : 72). The complementary molar ratio of TCA : GLY : SCCA is then 1 : 23 : 72 (or 0.043 : 1 : 3.310). The other molar ratios are listed in the table below:

| LCFAM : GLY : SCFAM | TCA : GLY : SCCA |
|---|---|
| 1:3:9 | 1 :8;27 |
| or | or |
| 3:9:27 | 0.125 : 1 : 3.375 |
| 1:8:12 | 1:23:36 |
| or | or |
| 3:24:36 | 0.043 : 1 : 1.565 |
| 1:3:4.5 | 1 : 8 : 13.5 |
| or | or |
| 3:9:4.5 | 0.125 : 1 : 1.688 |

Since it is preferred that the acetoglyceride composition comprises a relatively large number of free hydroxy groups, i.e. that the molar ratio of (i) the sum of long-chain fatty acid moieties and short chain carboxylic acid moieties and (ii) the glycerol moieties does not exceed 2.9 : 1, preferably does not exceed 2.7 : 1. The corresponding molar ratios of TCA : GLY : SCCA can easily be calculated by the person skilled in the art. Graphically this is shown in the figure below.

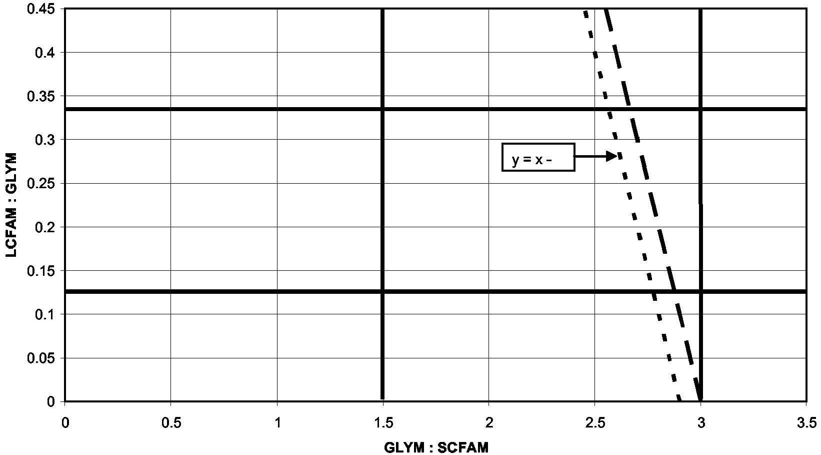

As described above, the esterification catalyst is preferably an acidic catalyst having a pKₐ of 2 - 4 and is preferably selected from the group consisting of phosphoric acid and C₁ - C₁₀ hydrocarbyl sulphonic acids, preferably p-toluenesulphonic acid and methanesulphonic acid, and more preferably methanesulphonic acid. In addition, step (c) is preferably carried out at a temperature of 120 - 150°C. Preferred embodiments of steps (d) and (e) are described above.

### EXAMPLES

For the analysis of the samples prepared during the examples to be given below, gas chromatographic analyses were performed on a TraceGC gas chromatograph equipped with an AS2000 autosampler (ThermoQuest), a flame ionisation detector and a capillary column. Helium has been used as a carrier gas at a flow rate of 10 mL/min. The oven temperature was programmed with an initial hold at 110°C for 1 min, 110 - 225°C at a rate of 25°C/min, 225 - 350°C at a rate of 3°C/min and a final hold at 350°C of 5 min.

The percentage composition was determined from GC peak areas calculated by the EZChrom Elite v 2.8 software package without any correction factors.

### EXAMPLE 1

In this example, distilled monoglycerides (90%) are used as the starting material. They were allowed to react with acetic anhydride and acetic acid and the latter reaction was carried out without a catalyst or with methanesulphonic acid as catalyst. A 2 L pressure vessel with steam jacket was used for the reaction.

Comparative Experiment 1. An amount of 1000 g (2.8 mole) Dimodan HR (Danisco Ingredients, Braband, Denmark), a distilled monoacyl glyceride (monoacyl glyceride content 90%), was mixed with 363 mL (3,91 mole) acetic anhydride and heated to 150°C in the reaction vessel at a pressure of 4.5 bar nitrogen for a period of 5.5 hours. After this period, the pressure is released so that the acetic acid and water formed are vented off. This causes the temperature to drop to 70°C. Analysis of the reaction product shows this to consist for about 90% of acetoglycerides [mixture of monoacetyl and diacetyl monoacyl glycerides with small amounts of monoacetyl diacyl and diacylglycerides] with an acetylation degree of 69% [the acetylation degree is determined by reacting a sample with an excess acetic acid anhydride followed by subsequent hydrolysis of any excess acetic acid anhydride to acetic acid and titration of the acetic acid formed]. The amounts of free fatty acids (1%) and acetins (2%) were negligible.

Comparative Experiment 2. In the next experiment, the same amount of monoacyl glyceride was mixed with 480 mL (8.4 mole) of glacial acetic acid and the mixture was allowed to react at 150°C for 5.5 hours. Some esterification had taken place but the degree of acetylation of the monoacyl glycerides was found to be only 35%.

Comparative Experiment 3. In the third experiment in this example, the same amounts of monloacyl glycerides and acetic acid were used but as a catalyst, 0.06 mole of methanesulphonic acid was included in the reaction mixture. The reaction temperature was kept at 130°C for a period of 6 hours. Under these reaction conditions, extensive interesterification was observed in that the reaction product contained only 51% acetoglycerides with an acetylation degree of 78%, 26% monoacetyl diacylglycerides, 9% free fatty acids and 13% acetins. Apparently, the presence of the catalyst causes extensive ester interchange in that the monoacyl glycerides are partially converted into acetins by loosing their long-chain fatty acid and also partially converted into diacyl glycerides by binding a long-chain fatty acid. This experiment also demonstrates that a higher glycerol moiety to long chain fatty acid moiety ratio is needed for an acetoglyceride product with a low content of manoacetyl diacyl glycerides.

### EXAMPLE 2

The experiments in this example were carried out in a 250 mL glass flask provided with a magnetic stirrer and fitted with a Vigreux column. The reagents were added to the flask and heated under reflux conditions while being agitated.

Comparative Experiment 1. By using glycerol, glacial acetic acid and free fatty acids (a mixture of palmitic and stearic acid) in a molar ratio of 1:1.4:1 and adding 0.05 molar sulphuric acid, rapid esterification was observed. After 3.5 hours all fatty acids had been esterified. However, the low glycerol content of the reaction mixture with respect to fatty acids caused the acetoglyceride fraction obtained after water washing to contain 38% monoacetyl diacyl glycerides and diacyl glycerides.

Experiment 2. Therefore the molar ratio was altered to 1:1.5:0.33 and behenic acid was used instead of the palmitic/stearic acid mixture. Instead of the sulphuric acidic catalyst, 0.06 mole of methanesulphonic acid was used After 6 hours under reflux conditions the water and acetic acid present in the reaction mixture were allowed to evaporate and the resulting mixture was washed with water. Subsequent GLC analysis showed the washed product to contain 76% of acetoglycerides with a degree of acetylation of 54 %, only 5% of monoacetyl diacyl glycerides and 14% free behenic acid and 5% of acetins which latter two compounds could have been easily removed during a steam stripping deodorisation treatment.

Comparative Experiment 3. When using even more glycerol and less behenic acid in molar ratio of 1:3:0.25, the composition of the product from which the catalyst had been removed by water washing was 25% acetoglycerides with a degree of acetylation of 63%, only 2% of monoacetyl diacyl glycerides and 33% free fatty acids and 40% acetins.

### COMPARATIVE EXAMPLE 3

In this example a 3 L three-necked flask was used that was fitted with a Snyder column. When glycerol, acetic acid and triglycerides were mixed in a molar ratio of 1:3.2:0.09 and heated under reflux in the presence of methanesulphonic acid, a reaction product resulted that after catalyst removal consisted of 38% acetoglycerides with a degree of acetylation of 73%, only 2% of monoacetyl diacyl glycerides, a trace of free fatty acids and 60% acetins.

It was noted that when aqueous acetic acid was used, the triglycerides present hardly reacted. Only after the water present had been removed by distillation did the triglycerides participate in the reaction. The removal of the water also caused the free fatty acid content of the reaction product to be reduced.

## Claims

1. A process for preparing an acetoglyceride composition comprising a high amount of monoacetyl monoacyl glycerides comprising the steps of:
(a) providing a reaction mixture comprising long chain fatty acid moieties, glycerol moieties and short chain carboxylic acid moieties;
(b) adding a catalyst;
(c) allowing this reaction mixture to approach or reach equilibrium and optionally shift the position of this equilibrium by removing the most volatile component or components from said reaction mixture;
(d) inactivating and/or removing the catalyst from the reaction mixture;
(e) purifying the reaction mixture;
wherein the purification step (e) comprises a separation of said reaction mixture into an acetoglyceride fraction and one or more non-acetoglyceride fractions and that at least one of those non-acetoglyceride fractions is recycled by being incorporated into the reaction mixture provided in step (a);
wherein the molar ratio of the long-chain fatty acid moieties and the glycerol moieties in the reaction mixture provided in step (a) is between 1:8 and 1:3; wherein the molar ratio of the short chain carboxylic acid moieties and the glycerol moieties in the reaction mixture provided in step (a) is between 1.5:1 and 3:1; and
wherein the molar ratio of (i) the sum of long-chain fatty acid moieties and short chain carboxylic acid moieties and (ii) the glycerol moieties does not exceed 2.9 : 1;
the long chain fatty acid moieties being acyl moieties, wherein the fatty acid moieties have a linear, saturated or unsaturated, C₁₄ - C₂₄ alkyl chain;
the short chain carboxylic acid moieties being acetyl groups;
the acetoglyceride comprising the following group of compounds: monoacetyl diacyl glycerides, diacetyl monoacyl glycerides and monoacetyl monoacyl glycerides;
the monoacetyl monoacyl glycerides being glycerol esters having one acyl group, one acetyl group and one free hydroxy group;
the monoacetyl diacyl glycerides being glycerol esters having two acyl groups and one acetyl group;
the diacetyl monoacyl glycerides being glycerol esters having one acyl group and two acetyl groups;
the monoacetyl monoacyl glycerides and diacetyl monoacyl glycerides being acetoglycerides having one long chain fatty acid moiety; and
the monoacetyl diacyl glycerides being glycerides having two long chain fatty acid moieties.

2. Process according to claim 1, wherein the catalyst added in step (b) is a basic catalyst.

3. Process according to claim 2, wherein the basic catalyst is selected from the group consisting of alkali metals, their hydrides, amides and alcoholates.

4. Process according to claim 2 or claim 3, wherein the basic catalyst is selected from the group consisting of sodium or potassium methanolate or ethanolate.

5. Process according to any one of claims 2 - 4, wherein step (c) is carried out at a temperature within the range of 70 - 100°C.

6. Process according to any one of claims 2 - 5, wherein the reaction mixture is substantially free from free acids and water.

7. Process according to any one of claims 2 - 6, wherein the reaction mixture comprising long chain fatty acid moieties, glycerol moieties and short chain carboxylic acid moieties is essentially free from components that are capable of inactivating the catalyst.

8. Process according to claim 1, wherein the catalyst added in step (b) is an acidic catalyst.

9. Process according to claim 8, wherein the acidic catalyst has a pKₐ in the range of 2-4.

10. Process according to claim 8 or claim 9, wherein the acidic catalyst is selected from the group consisting of C₁ - C₁₀ hydrocarbyl sulphonic acids.

11. Process according to claim 10, wherein the acidic catalyst is p-toluene sulphonic acid or methane sulphonic acid.

12. Process according to any one of claims 8 - 11, wherein step (c) is carried out at a temperature within the range of 120 - 150°C.

13. Process according to any one of claims 8 - 12, wherein water formed is removed from the reaction mixture by distillation.

14. Process according to any one of claims 1 - 13, wherein the catalyst is inactivated and/or removed by washing the reaction mixture with water.

15. Process according to any one of claims 1 - 14, wherein the separation during step (e) comprises a washing step with water.

16. Process according to claim 15, wherein the water comprises 10 - 30 wt.% of free short chain fatty acid, preferably acetic acid.

17. Process according to any one of claims 1 - 14, wherein the separation during step (e) comprises a steam stripping step.

## Patentansprüche

1. Verfahren zum Herstellen einer Acetoglyceridzusammensetzung umfassend eine hohe Menge von Monoacetyl-monoacyl-glyceriden umfassend die Schritte:
(a) Bereitstellen einer Reaktionsmischung umfassend langkettige Fettsäureeinheiten, Glycerineinheiten und kurzkettige Carbonsäureeinheiten;
(b) Zugeben eines Katalysators;
(c) Ermöglichen, dass diese Reaktionsmischung sich dem Gleichgewicht nähert oder es erreicht und gegebenenfalls die Lage dieses Gleichgewichts durch Entfernen der flüchtigsten Komponente oder Komponenten aus der Reaktionsmischung verschoben wird;
(d) Inaktivieren und/oder Entfernen des Katalysators aus der Reaktionsmischung;
(e) Reinigen der Reaktionsmischung;
wobei der Reinigungsschritt (e) eine Trennung der Reaktionsmischung in eine Acetoglyceridfraktion und eine oder mehrere Nicht-Acetoglyceridfraktionen umfasst und dass wenigstens eine von diesen Nicht-Acetoglyceridfraktionen recycliert wird, indem sie in die in Schritt (a) bereitgestellte Reaktionsmischung aufgenommen wird;
wobei das Molverhältnis der langkettigen Fettsäureeinheiten und der Glycerineinheiten in der in Schritt (a) bereitgestellten Reaktionsmischung zwischen 1:8 und 1:3 liegt;
wobei das Molverhältnis der kurzkettigen Carbonsäureeinheiten und der Glycerineinheiten in der in Schritt (a) bereitgestellten Reaktionsmischung zwischen 1.5:1 und 3:1 liegt; und wobei das Molverhältnis von (i) der Summe von langkettigen Fettsäureeinheiten und kurzkettigen Carbonsäureeinheiten und (ii) den Glycerineinheiten 2.9:1 nicht überschreitet; wobei die langkettigen Fettsäureeinheiten Acyleinheiten sind, wobei die Fettsäureeinheiten eine lineare, gesättigte oder ungesättigte C₁₄-C₂₄-Alkylkette aufweisen;
wobei die kurzkettigen Carbonsäureeinheiten Acetylgruppen sind;
wobei das Acetoglycerid die folgende Gruppe von Verbindungen umfasst: Monoacetyl-diacyl-glyceride, Diacetyl-monoacyl-glyceride und Monoacetyl-monoacyl-glyceride; wobei die Monoacetyl-monoacyl-glyceride Glycerinester mit einer Acylgruppe, einer Acetylgruppe und einer freien Hydroxygruppe sind;
wobei die Monoacetyl-diacyl-glyceride Glycerinester mit zwei Acylgruppen und einer Acetylgruppe sind;
wobei die Diacetyl-monoacyl-glyceride Glycerinester mit einer Acylgruppe und zwei Acetylgruppen sind;
wobei die Monoacetyl-monoacyl-glyceride und Diacetyl-monoacyl-glyceride Acetoglyceride mit einer langkettigen Fettsäureeinheit sind; und
wobei die Monoacetyl-diacyl-glyceride Glyceride mit zwei langkettigen Fettsäureeinheiten sind.

2. Verfahren nach Anspruch 1, wobei der in Schritt (b) zugegebene Katalysator ein basischer Katalysator ist.

3. Verfahren nach Anspruch 2, wobei der basische Katalysator aus der Gruppe bestehend aus Alkalimetallen, ihren Hydriden, Amiden und Alkoholaten ausgewählt ist.

4. Verfahren nach Anspruch 2 oder Anspruch 3, wobei der basische Katalysator aus der Gruppe bestehend aus Natrium- oder Kaliummethanolat oder -ethanolat ausgewählt ist.

5. Verfahren nach einem der Ansprüche 2 - 4, wobei Schritt (c) bei einer Temperatur in dem Bereich von 70 - 100 °C durchgeführt wird.

6. Verfahren nach einem der Ansprüche 2 - 5, wobei die Reaktionsmischung im Wesentlichen frei von freien Säuren und Wasser ist.

7. Verfahren nach einem der Ansprüche 2 - 6, wobei die Reaktionsmischung, die langkettige Fettsäureeinheiten, Glycerineinheiten und kurzkettige Carbonsäureeinheiten umfasst, im Wesentlichen frei von Komponenten ist welche imstande sind den Katalysator zu inaktivieren.

8. Verfahren nach Anspruch 1, wobei der in Schritt (b) zugegebene Katalysator ein saurer Katalysator ist.

9. Verfahren nach Anspruch 8, wobei der saure Katalysator einen pKₐ im Bereich von 2 - 4 aufweist.

10. Verfahren nach Anspruch 8 oder Anspruch 9, wobei der saure Katalysator aus der Gruppe bestehend aus C₁-C₁₀-Hydrocarbyl-sulfonsäuren ausgewählt ist.

11. Verfahren nach Anspruch 10, wobei der saure Katalysator p-Toluolsulfonsäure oder Methansulfonsäure ist.

12. Verfahren nach einem der Ansprüche 8 - 11, wobei Schritt (c) bei einer Temperatur in dem Bereich von 120 - 150 °C durchgeführt wird.

13. Verfahren nach einem der Ansprüche 8 - 12, wobei gebildetes Wasser durch Destillation aus der Reaktionsmischung entfernt wird.

14. Verfahren nach einem der Ansprüche 1 - 13, wobei der Katalysator durch Waschen der Reaktionsmischung mit Wasser inaktiviert und/oder entfernt wird.

15. Verfahren nach einem der Ansprüche 1 - 14, wobei die Trennung während Schritt (e) einen Waschschritt mit Wasser umfasst.

16. Verfahren nach Anspruch 15, wobei das Wasser 10 - 30 Gew.-% freie kurzkettige Fettsäure, vorzugsweise Essigsäure umfasst.

17. Verfahren nach einem der Ansprüche 1 - 14, wobei die Trennung während Schritt (e) einen Wasserdampfdestillationsschritt umfasst.

## Revendications

1. Procédé de préparation d'une composition d'acétoglycéride comprenant une grande quantité de monoacétyl monoacyl glycérides comprenant les étapes suivantes:
(a) fournir un mélange réactionnel comprenant des fractions acide gras à chaîne longue, des fractions glycérol et des fractions acide carboxylique à chaîne courte;
(b) ajouter un catalyseur;
(c) laisser ce mélange réactionnel approcher ou atteindre l'équilibre et facultativement décaler la position de cet équilibre en enlevant le composant ou les composants les plus volatiles du mélange réactionnel;
(d) inactiver et/ou enlever le catalyseur du mélange réactionnel;
(e) purifier le mélange réactionnel;
dans lequel l'étape de purification (e) comprend une séparation dudit mélange réactionnel en une fraction acétoglycéride et une ou plusieurs fractions non acétoglycéride et qu'au moins une de ces fractions non acétoglycéride sont recyclées par incorporation dans le mélange réactionnel fourni à l'étape (a);
dans lequel le rapport molaire des fractions acide gras à chaîne longue sur les fractions glycérol dans le mélange réactionnel fourni à l'étape (a) est compris entre 1:8 et 1:3;
dans lequel le rapport molaire des fractions acide carboxylique à chaîne courte sur les fractions glycérol dans le mélange réactionnel fourni à l'étape (a) est compris entre 1.5:1 et 3:1; et
dans lequel le rapport molaire de (i) la somme des fractions acide gras à chaîne longue et des fractions acide carboxylique à chaîne courte sur (ii) les fractions glycérol n'excède pas 2.9/1;
les fractions acide gras à longue chaîne étant des fractions acyle, dans lequel les fractions acide gras ont une chaîne alkyle en C₁₄-C₂₄ linéaire saturée ou insaturée;
les fractions acide carboxylique à chaîne courte étant des groupes acétyle; l'acétoglycéride comprenant le groupe de composés suivant: les monoacétyl diacyl glycérides, les diacétyl monoacyl glycérides et les monoacétyl monoacyl glycérides; les monoacétyl monoacyl glycérides étant des esters de glycérol ayant un groupe acyle, un groupe acétyle et un groupe hydroxy libre;
les monoacétyl diacyl glycérides étant des esters de glycérol ayant deux groupes acyle et un groupe acétyle;
les diacétyl monoacyl glycérides étant des esters de glycérol ayant un groupe acyle et deux groupes acétyle;
les monoacétyl monoacyl glycérides et les diacétyl monoacyl glycérides étant des acétoglycérides ayant une fraction acide gras à chaîne longue; et
les monoacétyl diacyl glycérides étant des glycérides ayant deux fractions acide gras à chaîne longue.

2. Procédé selon la revendication 1, dans lequel le catalyseur ajouté dans l'étape (b) est un catalyseur basique.

3. Procédé selon la revendication 2, dans lequel le catalyseur basique est sélectionné dans le groupe constitué des métaux alcalins, de leurs hydrures, amides et alcoolates.

4. Procédé selon la revendication 2 ou la revendication 3, dans lequel le catalyseur basique est sélectionné dans le groupe constitué du méthanolate ou de l'éthanolate de sodium ou de potassium.

5. Procédé selon l'une quelconque des revendications 2-4, dans lequel l'étape (c) est réalisée à une température dans la plage de 70 à 100°C.

6. Procédé selon l'une quelconque des revendications 2-5, dans lequel le mélange réactionnel est sensiblement dépourvu d'acides libres et d'eau.

7. Procédé selon l'une quelconque des revendications 2-6, dans lequel le mélange réactionnel comprenant des fractions acides gras à chaîne longue, des fractions glycérol et des fractions acide carboxylique à chaîne courte est sensiblement dépourvu de composants qui sont capables d'inactiver le catalyseur.

8. Procédé selon la revendication 1, dans lequel le catalyseur ajouté dans l'étape (b) est un catalyseur acide.

9. Procédé selon la revendication 8, dans lequel le catalyseur acide a un pKa dans la plage de 2-4.

10. Procédé selon la revendication 8 ou la revendication 9, dans lequel le catalyseur acide est sélectionné dans le groupe constitué de C₁-C₁₀ acides hydrocarbyl sulfoniques.

11. Procédé selon la revendication 10, dans lequel le catalyseur acide est l'acide p-toluène sulfonique ou l'acide méthane sulfonique.

12. Procédé selon l'une quelconque des revendications 8-11, dans lequel l'étape (c) est réalisée à une température dans la plage de 120-150°C.

13. Procédé selon l'une quelconque des revendications 8-12, dans lequel l'eau formée est éliminée du mélange réactionnel par distillation.

14. Procédé selon l'une quelconque des revendications 1-13, dans lequel le catalyseur est inactivé et/ou éliminé par lavage du mélange réactionnel avec de l'eau.

15. Procédé selon l'une quelconque des revendications 1-14, dans lequel la séparation au cours de l'étape (e) comprend une étape de lavage avec de l'eau.

16. Procédé selon la revendication 15, dans lequel l'eau comprend de 10-30 % en poids d'acide gras à chaine courte libre, de préférence d'acide acétique.

17. Procédé selon l'une quelconque des revendications 1-14, dans lequel la séparation durant l'étape (e) comprend une étape d'extraction à la vapeur.
